# EUROPEAN PATENT APPLICATION

(11) **EP 2 138 196 A1**
(43) Date of publication of application: **30.12.2009**
(21) Application number: 09169169.1
(22) Date of filing: 20.03.2007
(51) Int. Cl.: A61L 27/38, C12N 5/06

(54) **Methods to maintain, improve and restore the cartilage phenotype of chondrocytes**

(30) Priority: 20.03.2006 US 783986 P
(62) Divisional of application: 07723416.9
(71) Applicant: TIGENIX N.V., 3001 Leuven (BE)
(72) Inventor: Knipper, Andreas, 41470, Neuss (DE); Muir-Mcleod, Paula, Dalgety Bay, Fife KY11 9FN (GB)
(74) Representative: Bird, William Edward

(57) **Abstract**

The present invention relates to regulatory cells, which are capable of restoring, maintaining or improving the stable cartilage phenotype of expanded and passaged chondrocytes. These regulatory cells are also capable of directing precursor and stem cells into the chondrogenic lineage. An enriched population of regulatory cells can be obtained by harvesting the non-adherent cells in the culture medium of a monolayer culture of P0 chondrocytes.

## Description

### FIELD OF THE INVENTION

The present invention relates to the field of chondrocyte expansion and cartilage repair. The invention also relates to regulatory cell populations obtainable from cartilage, which can be used to maintain, improve or restore the cartilage phenotype of chondrogenic cells such as chondrocytes or chondrocyte precursor cells.

### BACKGROUND OF THE INVENTION

Both articular and meniscal cartilage play an important role in the mechanics of normal joint movement. Articular cartilage covers the ends of these bones at the knee joint. It is composed of sparsely distributed cells (chondrocytes) embedded in an extracellular matrix composed of a three-dimensional mesh of collagens, various proteins/glycoproteins and large water-retaining proteoglycans which carry negatively charged polysaccharides called glycosaminoglycans. Meniscal cartilage is fibrocartilage characterized by the presence of cells in lacunae aligned in rows and bundles of collagen fibers in the matrix. Damage to either articular and/or meniscal cartilage is a common condition affecting the joints of millions of people. This damage is complicated by the poor regenerative capacity of adult articular cartilage and the disability and pain that accompanies this damage. Recently, several models of cartilage repair have been proposed. In one such model, the "Autologous Chondrocyte Transfer" technique (ACT), cells are retrieved from healthy articular cartilage, allowed to proliferate in culture in order to supply adequate cell numbers and are re-implanted into articular or meniscal cartilage defects [Brittberg et al. (1994) N Engl. J Med. 331, 889-895]. Cell expansion is necessary to obtain from a small cartilage biopsy sufficient cells to repair the cartilage defect. The ACT technique has limitations as chondrocytes expanded in monolayer culture have a propensity to dedifferentiate with increasing passage number. Such dedifferentiation will lead to the synthesis of a matrix with biochemical and biomechanical properties different to those found in the tissue of origin. Optimised cell culture conditions and limited passage numbers are needed to keep the phenotypic traits of chondrocytes including their capacity to form stable hyaline cartilage in vivo, resistant to vascular invasion and endochondral bone formation. All currently applied ACT procedures use a cellular therapeutic that is composed of an expanded chondrocyte population obtained from an original biopsy by cell cultivation methods. Irrefutably, these populations comprise variable numbers of cells that have retained their phenotypic stability and of dedifferentiated cells unable to redifferentiate towards stable articular cartilage-forming cells. This issue is underscored by the large variability in a clinical study in the quality of the repair tissue ranging from hyaline-like cartilage over fibrocartilage to no sign of repair [Peterson et al. (2000) Clin Orthop Relat Res. 374, 212-234]. This indicates that cell expansion to sufficiently large number of chondrocytes is not sufficient as such to perform a successful transplantation.

Different approaches have been used to improve the quality of chondrocytes or to use alternative sources of chondrocytes. Luyten et al. (WO0124833) describe the use of molecular markers as a quality control for expanded and passaged chondrocytes. The use of these markers allows to determine the number of passages that a chondrocyte population can undergo without significant loss of cartilage forming capacity upon transplantation. Different attempts have been made to increase the outcome of ACT by adding different matrix components to the chondrocytes, such as perlecan [French et al. (1999) J. Cell. Biol. 145, 1103-1115].

The surrounding matrix has also been reported to have an effect on the regenerative capacity of chondrocytes. Graff et al. [2003, Biotechnol. Bioeng. 82, 457-464] describe a cell population characterized by the presence of a native pericellular matrix ("chondrons") obtained from cartilage and document improved cartilage-forming properties of these cells compared to chondrocytes that have rebuilt their pericellular membrane *in vitro.*

Cartilage biopsies have been described to comprise, apart from the typical chondrocytes, a smaller fraction of cells with a differing morphology and behaviour. Kamil et al. [2004, Tissue Eng. 10, 139-144] describe the recovery from cartilage biopsies of a population of cells which are normally discarded ("floating cells") and describe how these cells can nevertheless be expanded in order to obtain a higher yield in expansion protocols. This report suggest that other fractions than the classical adherent chondrocyte fraction of cartilage biopsies may also be useful as a source of cartilage-forming cells.

Although methods are available to expand chondrocytes and to assay their quality, it is as yet not possible to obtain a sufficient amount of phenotypically stable chondrocytes within a limited number of passages, to improve the properties of low-quality chondrocytes or to restore the properties of cells after improper handling (e.g. excessive incubation or expansion). In view of the requirement to produce a consistent amount of high quality cells for ACT, there is a need for methods which make it possible to ensure this consistency, independent of the quality and number of the chondrocyte cells present in the biopsy.

### SUMMARY OF THE INVENTION

The present invention relates to a population of cells obtainable from cartilage which, upon combination with a population of cells with chondrogenic capacity, has a regulatory effect thereon, whereby chondrocyte phenotypic stability is increased, induced or restored. This regulatory cell population is naturally present as a fraction of a freshly isolated cartilage biopsy but can be enriched and/or separated based on its physical and/or physiological properties. It has been established that this cell population retains its regulatory capacity when frozen, making it possible to store the regulatory cell population (either in enriched or non-enriched form), e.g. during the period necessary to expand mature chondrocytes to a sufficient large number required for transplantation. Once a sufficient number of chondrocytes is obtained, the chondrocyte cell population is combined with a population of regulatory cells, and the combined population is ready for transplantation.

Moreover it has been found that this regulatory cell population can be separated out from the chondrocytes of a cartilage biopsy by harvesting, within a certain time, e.g. after about 2 to 5 days after seeding and/or when the adhering cells have reached at least 30% confluency, the non-adhering fraction of cells from the cultivation recipient.

In one aspect, the present invention provides methods for enriching the regulatory cell population of the present invention. More particularly, the invention provides methods for enriching a population of regulatory cells from an isolated cartilage sample, said method comprising the steps of (a) mechanically and/or enzymatically treating an isolated cartilage sample to obtain individual cells; (b) transferring the so-obtained individual cells to a cell cultivation recipient to allow the expansion of chondrocytes in a monolayer; (c) maintaining the cell cultivation recipient in adequate cell cultivation conditions, thereby obtaining a population of adherent cells and a supernatant comprising a population of non-adherent cells; (d) collecting from the cell cultivation recipient, after at least 2 days and/or when the adherent cells have reached at least about 30% confluency, the supernatant comprising a population of non-adherent cells; (e) collecting from the supernatant, the population of non-adherent cells, corresponding to the regulatory cell population of the present invention.

According to one embodiment, the cartilage sample used in the enrichment method of the present invention is a sample of articular cartilage or of meniscal cartilage.

Typically, the enrichment methods of the present invention will start from a cartilage sample which is enzymatically treated using collagenase A.

In a further aspect, the present invention provides an enriched regulatory cell population which is a population of non-adhering, non-passaged regulatory cells obtainable by the methods of enrichment described herein.

In yet a further aspect, the present invention provides different uses of the enriched regulatory cell population of the present invention.

According to a first embodiment, the enriched regulatory cell population obtainable by the methods of enrichment of the invention is provided for use as a medicament.

More specifically, the present invention provides methods for the treatment of cartilage defects comprising administering to a patient with a cartilage defect, a population of enriched regulatory cells of the present invention. Accordingly, the present invention provides pharmaceutical compositions comprising the enriched regulatory cell population of the present invention.

More particularly, the invention provides an improved method of treatment involving the introduction of microfractures into articular cartilage defects. Provision of microfractures in a defect allows the entry of stem cells of underlying bone into articular cartilage defects. Hereafter, *in vivo* cartilage reconstitution is obtained by applying a cellular therapeutics, which, according to the present invention comprises a cell population with regulatory function. In this reconstitution process the mesenchymal stem cells are directed into the chondrogenic lineage by the regulatory cell population of the invention.

According to a further embodiment, the enriched regulatory cell population is used for improving, maintaining or restoring the chondrocyte phenotypic stability of an expanded or passaged chondrocyte population *in vitro.*

According to yet a further embodiment of the invention, the enriched regulatory cell population is used for differentiating mesenchymal stem cells into the chondrogenic lineage *in vitro.*

In yet a further aspect, the present invention provides a composition comprising the regulatory cell population (either in enriched form or as present in freshly isolated cartilage cells) one or more different cell populations.

More particularly, the invention provides a composition comprising a combination of two or more different cell populations comprising:
- a cell population comprising regulatory cells which is either (i) a population of freshly isolated cartilage cells obtained from a cartilage biopsy or (ii) a population of enriched regulatory cells obtainable by the collection of non-adherent cells from the supernatant of a P0 culture of freshly isolated cartilage cells obtained from a cartilage biopsy; and
- one or more cell populations selected from the group consisting of a passaged chondrocyte cell population, a population of mesenchymal stem cells, and a population of chondrocyte precursor cells.

More specifically, the present invention provides compositions which comprise combinations of two or more different cell populations comprising a population of enriched regulatory cells, whereby the relative amount of the enriched regulatory cells is generally within the range of 1 to 75 % of the total number of cells in the combined composition.

According to a particular embodiment, the population comprising regulatory cells which is present in the combined compositions of the present invention is obtained from articular cartilage or meniscal cartilage.

The invention further provides different applications of the combination of the regulatory cell population (either in enriched form or as present in freshly isolated cartilage cells) with different cell populations.

More specifically the combined cell population comprising a cell population of regulatory cells according to the invention is provided as a medicament. Accordingly the present invention provides pharmaceutical compositions comprising the combined cell populations described herein. The combined cell populations can also be provided in a scaffold for therapeutic use.

The present invention provides the use of the combination of the (optionally enriched) regulatory cell population of the present invention with a second cell population in the preparation of a cellular therapeutic for the treatment of cartilage defects. Accordingly methods of treatment for cartilage defects are provided comprising administering to a patient having a cartilage defect, a composition comprising the regulatory cell population of the present invention and one or more other cell populations.

The use of the regulatory cell population in combination with other cell populations allows the use of e.g. chondrocytes of higher passage numbers for implantation. Furthermore, the methods of the present invention make it possible to generate larger amounts of chondrocytes by repeated passaging. The eventual decrease in cartilage phenotype upon extensive passaging can be restored by addition of the regulatory cell population of the present invention.

The use of the cell population of the present invention thus enables the cellular treatment of larger defects, since higher amounts of chondrocytes with a stable phenotype can be obtained. Alternatively, independent of the number of chondrocytes available, the quality thereof can be improved by using the regulatory cell population of the present invention.

The use of the regulatory cell population of the present invention ensures an improvement of commonly used ACT procedures.

More particularly, the present invention provides methods for preparing a population of cells for ACT transplantation, comprising the steps of:
a) mechanically and/or enzymatically treating an isolated cartilage sample to obtain individual cells,
b) transferring the individual cells so obtained to a cell cultivation recipient to allow the expansion of chondrocytes in a monolayer,
c) maintaining the cultivation recipient in adequate cell cultivation conditions, thereby obtaining a population of adherent cells and a supernatant comprising a population of non-adherent cells,
d) collecting from the cultivation recipient, after at least 2 days or upon at least about 30% confluency of the adherent cells in the cultivation recipient, the supernatant comprising a population of non-adherent cells,
e) collecting said non-adherent cells from said supernatant,
f) combining the so-obtained population of non-adherent cells with a population of chondrogenic cells, which is not a population of enriched regulatory cells.
   According to one embodiment of the methods for preparing a population of cells for ACT transplantation, the population of chondrogenic cells is a population of mature chondrocytes obtained from the same or a different isolated cartilage sample. More particularly, the method further comprises the steps of
g) expanding and passaging the population of adherent cells obtained in step (c),
h) collecting the expanded and passaged population of adherent cells,
i) in step (f), combining the population of non-adherent cells obtained in step (e) with the population of expanded and passaged population of adherent cells obtained in step (h).

Typically, in the methods of the present invention, the enzymatic treatment in step (a) is performed with collagenase A.

In specific embodiments of the methods of the present invention the isolated cartilage sample is a meniscal cartilage sample. Optionally, the population of chondrogenic cells originates from a meniscal cartilage biopsy. Alternatively, the population of chondrogenic cells is a population of mesenchymal stem cells.

Further embodiments of the methods for preparing a population of cells for ACT transplantation of the present invention, further comprise the step of seeding the combined populations obtained in step (f) or (i) on a scaffold.

The invention thus relates to methods of improving *in vivo* cartilage reconstitution by applying a cellular therapeutics comprising a mixture or combination of an autologous or allogeneic regulatory chondrocyte population according to the invention and expanded autologous or even allogeneic chondrocytes with a fibroblastic morphology.

The invention further relates to methods of improving *in vivo* cartilage reconstitution by applying a cellular therapeutics comprising a mixture of the regulatory cell population according to the invention and mesenchymal stem cells.

The compositions comprising a combination of the cell population of the present invention and chondrocytes is capable of a faster cartilage formation *in vitro*. Accordingly, it is envisaged that implantation of a combination of both chondrocytes and the regulatory cell population of the present invention results in improved cartilage reconstitution when injected in vivo, which initiates a faster regenerative process and high quality cartilage formation.

Moreover, it was observed that cartilage, generated by the cell population of the present invention in pellet culture, demonstrated a fast formation of mechanically stable pellets, even when combined with dedifferentiated chondrocytes of higher passages. This not only provides more flexibility with regard to the chondrocyte population amenable for implantation, but also has implications for the impact on local chondrogenic cells. The ability to induce the stable chondrocyte phenotype in partly dedifferentiated cells indicates that the regulatory cells of the present invention are applicable of inducing cartilage formation e.g. in osteoarthritic conditions, where local chondrocytes are already affected by catabolic processes within the joint.

### DETAILED DESCRIPTION OF THE INVENTION

### Definitions:

The term "**chondrogenic**" when applied to a cell or a population refers to the inherent capacity of that cell or population to produce cartilage or to stimulate cartilage growth, under appropriate circumstances. Chondrogenic cells include chondrocytes and cells which themselves differentiate into chondrocytes, such as precursors committed to the osteochondral lineage.

A "**chondrogenic factor**" as used herein refers to a compound which promotes cartilage differentiation, such as certain growth factors, such as TGF-β.

The term "**chondrocyte phenotypic stability**" when referring to a cell population, refers to the ability of the cell population to produce cartilage *in vivo.* This ability can be tested by injecting a fraction of the cell population (at least about 1 - 20 x 10⁶ cells) in a mammal (*in vivo*), such as immune-deficient mice, and determining (in a time frame of about 3 weeks), the development of a cartilage implant without signs of vascular invasion or endochondral bone formation. A chondrocyte population that is phenotypically stable, is moreover characterized by the presence of markers of phenotypic stability as described in WO0124833. Chondrocyte phenotypic stability is gradually lost in mature chondrocytes upon passaging.

The term "**freshly isolated cells**" (FI) as used herein refers to the cell population obtained from a biopsy after tissue digestion. The term "**freshly isolated cartilage cells**" as used herein thus refers to the cell population directly obtained from a chondrocyte-containing tissue, such as cartilage, by digestion, without passaging. The freshly isolated cell population in the context of the present invention is used either directly (i.e. within 48-120 hours after isolation) or is frozen for later use. Alternatively, the features of the freshly isolated cell population can be maintained for a longer period of time under certain cultivation conditions e.g. by cultivation for a longer period of time at high density in ultra-low attachment plates in an incubator without passaging.

The term "**regulatory**" when relating to a cell or cell population refers to the capacity of the cell or cell population when contacted with another cell population, to influence chondrogenicity of that cell population, and/or to initiate chondrocyte expansion, and/or to influence the chondrocyte phenotypic stability of the other cell population.

The term "**enriched**" when referring to the regulatory cell population of the present invention relates to the fact that from the freshly isolated cells as obtained from a cartilage biopsy, the cells developing into mature chondrocytes have been removed, and thus that a higher percentage of regulatory cells is obtained as compared to the percentage of regulatory cells present in the total of freshly isolated cells. According to the present invention one particular way of enriching the regulatory cell population is based on their non-adhering properties.

The term "**non-adhering**", "**floating**", or "**non-attaching**" when used to refer to a cartilage-derived cell or cell population refers to the fact that the cells or cell population, when introduced into a cultivating recipient (such as a culture flask), remains in suspension and does not adhere to the surface of the recipient. More specifically, a non-adhering chondrocyte cell population (NAC) of the present invention is the cell population present in the supernatant of a chondrocyte monolayer of freshly isolated cartilage cells (i.e. during a first expansion under standard cultivating conditions), after at least two days or when the adherent cells have obtained at least 30% confluency.

When referring to "**mature chondrocytes**" in the present invention, it is intended to refer to the mature cells of cartilage tissue, which originate from chondroblasts and are capable of producing cartilage. Mature chondrocytes are obtained upon cultivation of a population of freshly isolated cells from cartilage. When allowed to attach to a matrix or support, such as upon cultivation in monolayer, the freshly isolated cells flatten out and obtain a polygonal or fibroblastic morphology, lose their pericellular matrix and become mature chondrocytes. A "**mature chondrocyte cell population**" as used herein thus refers to a population of cells obtained by cultivation of cartilage cells in monolayer, e.g. in culture flask, whereby the mature chondrocytes adhere to the surface and are passaged one or more times.

The term "**expanded**", when referring to a cell population obtained from a tissue, such as cartilage, indicates that the cell population has been placed under conditions whereby the number of cells has increased by proliferation. In its simplest version, expansion is performed by providing the cell population in a cultivation recipient with appropriate cultivation medium, optionally until confluency.

The "**first expansion**" of a cell population isolated from a tissue refers to the cultivation of the cell population prior to the first passaging. During this first expansion, a fraction of the freshly isolated cells will adhere to the surface of the flask and become mature chondrocytes (P0 mature chondrocytes), while a fraction of the freshly isolated cells will remain as non-adhering cells in the supernatant (non-adhering cell population), Typically, the first expansion will be about 15 days, depending on the density of the cells seeded. Upon passaging, the supernatant of the cultivated cells is removed and the adherent cells are detached from the culture recipient, divided and again transferred to a culture recipient with fresh medium (this stage corresponding to P1). The P1 cells again adhering to the cultivation flask can be further passaged. P1 cells or cells with a higher passage number are generally referred to herein as "**passaged cells**".

The term "**cartilage defect**" as used herein refers to any condition resulting from the loss or damage of cartilage. Most often, these occur in the joints, such as, but not limited to knee, elbow, ankle and are then commonly referred to as articular cartilage defects. Cartilage defects are also named after the proximal bone such as defects of the condyles of the femur, of the humerus etc. Loss/tear of fibrocartilage of the meniscus is also referred to as a meniscal defect.

The present invention is based on the observation that freshly isolated cells obtained from a cartilage biopsy comprise, besides a population of cells which will develop into traditional mature chondrocytes and which, when introduced into a culture flask, adheres to the surface of the flask and can be expanded, a population of non-adherent cells, which not only is phenotypically stable, but can act as a regulatory cell population. The regulatory activity of this cell population is apparent from the fact that it is capable of maintaining chondrocyte phenotypic stability of a chondrocyte population and of rescuing chondrocyte phenotypic stability of dedifferentiated cell populations. Thus, this cell population present within freshly isolated cells obtained from cartilage is of particular interest for cartilage production and repair. Accordingly, the present application provides for novel applications of freshly isolated cartilage cells, which make use of the features of the sub-population of regulatory cells therein. Moreover, the non-adherent feature of this regulatory cell-population provides for a method of enrichment of the regulatory cell population of the invention. Thus, while traditionally the non-adherent cell population obtained in the first expansion of a chondrocyte culture is discarded, the present invention provides for the use of this non-adherent cell population, not only as a chondrogenic cell population but as a regulatory population which is capable of significantly influencing the chondrocyte phenotypic stability of other cell populations.

It has been observed that the regulatory cell population of the present invention comprises cells with a pericellular matrix. Chondrocytes have a rounded or semi-rounded appearance when present in cartilage tissue. Digestion of the cartilage tissue destroys the native pericellular matrix present around the cells. During the first 48 hours of cultivation, the cells regain an, *albeit* different, pericellular matrix. When allowed to attach to a matrix or support, such as upon cultivation in monolayer, most of the freshly isolated cells flatten out and obtain a polygonal or fibroblastic morphology, again losing most of their pericellular matrix. The regulatory cell population of the present invention is characterized by the presence of cells with a rounded morphology, around which this regained pericellular matrix is maintained.

According to the present invention the regulatory cell population of the present invention is present in freshly isolated cells as obtained from every cartilage biopsy. Thus the present invention provides methods involving the use of this regulatory cell population, both as present in freshly isolated cells and as an enriched cell population, in cartilage production *in vitro* and *in vivo*. The invention further provides for methods for enriching a cell population with regulatory function.

The regulatory cell population of the present invention is, either as part of a freshly isolated cell population, or as an enriched regulatory cell population, typically obtained from an isolated cartilage sample. According to the present invention, the isolated cartilage is fibrocartilage and/or articular cartilage obtained from anywhere in the human or animal body. Most particularly locations in the body which are easily accessible for obtaining a cartilage biopsy are selected. If the isolation of the population of regulatory cells is performed in the context of providing cells for an ACT, the biopsy of cartilage ideally comes from a non-weightbearing area that has little contact with other bones, such as the intercondylar notch of the femur. Alternatively, the cartilage sample used in the method of the present invention is herniated spinal discs cartilage or meniscal cartilage (fibrocartilage).

The cartilage biopsy used for the generation of cells in the present invention can be obtained from young or old individuals, from healthy or diseased cartilage (e.g. osteoarthritic cartilage).

The first aspect of the present invention relates to a method for enriching the regulatory cell population of the present invention. In its most general form, the method comprises the following steps:
- mechanically and/or enzymatically treating an isolated cartilage sample to obtain a population of individual cells,
- transferring the population of individual cells so obtained to a cell cultivation recipient to allow the expansion of chondrocytes in a monolayer,
- maintaining the cultivation recipient under adequate cell cultivation conditions, and
- collecting from the cultivation recipient the supernatant comprising a population of non-adherent cells, corresponding to the regulatory cell population of the present invention.

According to the present invention, cultivation of freshly isolated cells from cartilage tissue provides a method for the physical separation of the cells into an 'adhering' population, which comprises essentially mature chondrocytes, and a non-adhering population, having regulatory features.

In a first step of the enrichment procedure according to the present invention, the isolated cartilage is treated so as to obtain a population of individual cells ('freshly isolated cartilage cells'). According to a particular embodiment, such treatment comprises an enzymatic treatment. Enzymes that are suitable for the digestion of cartilage biopsies include, but are not limited to, Collagenase NB4, Collagenase A or a mixture of dispase/collagenase II. In a particular embodiment of the present invention the cartilage biopsy is treated with Collagenase A. Other proteinases which are suitable for the disruption of cartilage include enzymes (either alone or in combination with trypsin) selected from the group consisting of aspartate proteinases like Cathepsin D, cysteine proteinases like Cathepsin B, L, S, K and calpains I and II, serine proteases like neutrophil elastase, Cathepsin G and Proteinase 3 and metalloproteinases like MMPs 1-20. The 'treatment' according to the present invention comprises the contacting of the isolated tissue with an amount of enzyme for a time period sufficient to allow digestion of the tissue into individual cells. Exemplary enzyme concentrations and time periods for digestion are described in the examples section herein and alternative treatments can be easily identified by the skilled person. In a particular embodiment of the invention Collagenase A is used in range between 0,1 and 0,3 % for a time period between 8 to 16 hours, more particularly 12 hours.

Alternatively, the cartilage biopsy can be treated by mechanical means to obtain a population of individual cells, such as by slow speed mechanical homogenisation as described in Poole et al. (1988) J. Orthop. Res 6, 408-419.

In the enrichment procedure according to the present invention, the individual cells obtained from a cartilage biopsy ('freshly isolated cartilage cells') are introduced into a 'cultivation recipient', in order to allow the separation of the 'adhering' and 'non-adhering' populations present therein. Suitable cultivation recipients for use in accordance with the present invention are the standard culture flasks used for the cultivation of matrix-dependent cell cultures (such as, but not limited to, FaIcon^{™} flasks from Becton Dickinson, TPP flasks from Techno Plastic Products, Greiner tissue culture treated flasks etc..). Most typically, these culture flasks will comprise an inner surface or flask base to which a surface treatment has been applied. Culture flasks of different shapes and sizes can be used, and these aspects are not critical, in the context of the present invention. The only limitation is that the flask be closed, i.e. that it is possible to maintain the cultivation medium above the adherent cell population for a time-period needed for the mature chondrocyte population to adhere to the bottom surface, so as to allow the physical separation of the adherent and non-adherent cells and the recovery of the non-adherent cells from the medium. According to a particular embodiment, the cultivation medium and conditions used for this step of the enrichment method of the invention is a standard cultivation medium for chondrocytes. Media used in the art for the expansion of chondrocytes typically comprise DMEM, HAMS/F12 or other cell cultivation media optionally supplemented with 5 to 15 % serum. Alternatively, serum-free media suitable for chondrocyte cultivation can be used. These media can comprise cocktails of vitamins, growth factors, hormones, sugars etc. According to a particular embodiment, DMEM medium with serum and antibiotics is used. The cell culture is standardly performed at 37°C with 5% CO₂, but it is expected that minor variations of these conditions will not significantly affect the adherence of the cell populations to the surface of the culture recipient according to the present invention. According to a particular embodiment of the invention, supernatant in the culture flask comprising the non-adherent cell population is to be maintained in the culture flask for a limited period of time, e.g. it is collected about 2 to 5 days after seeding or until the adherent cells attain at least about 20%, more particularly about 30% confluency. This will however be determined by the size of the flask and the number of cells originally introduced therein. In order for the culture of adherent cells to attain confluency within the culture flask within about two weeks in the first expansion, typically 5,000-20,000 freshly isolated cartilage cells are introduced into a flask of 175cm². Cultivation techniques of cartilage cells are well known to the skilled person.

The regulatory cell population of the present invention is **characterized in that** it is non-adhering, i.e. it does not adhere to the surface of a culture flask about 2 to 5 days after seeding of a culture flask with cells obtained from a cartilage biopsy or when adherent cells from a cartilage biopsy have reached about 30% confluency. According to the present invention, the regulatory population can be obtained by separating adhering and non-adhering cells in the first expansion of a freshly isolated cartilage cell population. Typical mature chondrocytes without a native pericellular membrane, present in a cell population obtained from a cartilage biopsy, adhere to the surface of a (pre-treated) cultivation recipient within a period of 1 week. This process of cell attachment is the basis of the method of enrichment for the regulatory cell population, which cell population is **characterized in that** it withstands the dedifferentiation and attachment process for a longer period of time. It will be understood that the moment of collection of the regulatory cell population in the enrichment process of the present invention can be less than 2 days or more than 5 days after seeding, but this will affect the efficiency of the enrichment process as the supernatant is likely to contain a higher fraction of mature chondrocytes which have either not yet adhered or are no longer adhering to the surface of the cultivation recipient, respectively.

In the enrichment procedure of the present invention, the non-adherent cell population is physically removed from the adherent cell population. According to an embodiment of the present invention, the non-adhering cell population is collected together with the supernatant from the culture recipient. More particularly, the retrieval of the non-adhering cell population from the culture recipient encompasses the steps of applying soft mechanical forces (e.g. slight tapping of the flask) to detach weakly-adhering cells and collecting the culture supernatant. The cells can be recovered from this supernatant by standard techniques such as, but not limited to, centrifugation (e.g. 1500 rpm for 10 minutes). Alternative methods include other separation methods such as filtration.

Typically, the enrichment procedures of the present invention result in the percentage of regulatory cells present in the population increasing with at least 10%, more particularly at least 20%, most particularly at least 30% compared to the percentage of regulatory cells present in the freshly isolated biopsy. Typically chondrocyte biopsies were found to contain between 5-20% or regulatory cells. While the nature of the cells immediately after recovery of the cells from the biopsy may be difficult to establish, this can also be determined after the mature chondrocytes have started to adhere to the surface of the cultivation flask. According to particular embodiments, the enriched regulatory cell population of the invention comprise at least 30%, more particularly at least 40%, most particularly between 50% and 95% regulatory, non-adhering cells.

According to a further embodiment, the adhering and non-adhering cell population are separated based on morphological and/or physiological features. Thus, the regulatory cell population of the present invention is characterized by a round cell morphology and a pericellular matrix, which, contrary to the pericellular matrix described for 'chondrons' by Graff et al. (2003, above), is not the native pericellular matrix, but one that has been regained by the cells in cultivation. The regulatory cell population of the present invention is further characterized by the expression of Collagen type II and Aggrecan. Alternative methods for isolating the regulatory population of the present invention thus include methods wherein the treatment of the cartilage biopsy to provide individual cells as described above, is followed by separation techniques to separate classical chondrocytes and regulatory cell population, e.g. by FACS analysis or other cell separation techniques.

While the present invention provides for methods for obtaining an enriched regulatory cell population from the biopsies described above, it is also noted that the freshly isolated cells obtained from these biopsies contain the regulatory cell population of the invention and can be used as such. Accordingly, the present invention envisages the use of freshly isolated cells both as a source of regulatory cells for enrichment or directly in the different applications of the regulatory cells of the present invention described below.

Thus, according to one embodiment of the present invention, the regulatory cell population is used in the different applications described herein, as present in a freshly isolated cell population. The freshly isolated cell population can be used directly, i.e. within 24 hours of its isolation from cartilage tissue, under standard cultivation conditions. It has further been established that the regulatory features of the regulatory cell population present within a freshly isolated cell population can be maintained *in vitro* for a longer period of time under certain cultivation conditions, such as keeping the cells in an incubator at high density in ultralow attachment plates (available from e.g. Corning). Alternatively, the freshly isolated cell population is frozen within 24 hours of its isolation, for later use. Methods for freezing and thawing of cell populations are known in the art.

According to one embodiment, freshly isolated cells obtained from a cartilage biopsy are split into two fractions, one of which is further expanded to increase the number of cells (using classical chondrocyte expansion techniques), and the other is frozen until expansion of the first fraction is completed, after which the thawed fraction of freshly isolated cells is combined with the expanded and passaged fraction of chondrocytes to create a combined cell population which can be used as a cellular therapeutic with increased phenotypic stability according to the invention.

According to another embodiment of the present invention, the regulatory cell population is used in the different applications described herein as an enriched regulatory cell population, obtainable by the methods described herein. Again, the enriched regulatory cell population can either be used directly or frozen for later use.

Further aspects of the present invention thus provide different applications of the regulatory cell population of the present invention.

One aspect of the invention provides combinations of a) the regulatory cell population of the present invention - either in 'enriched' or in non-enriched, i.e. as present within a freshly isolated cartilage cell population, form - and b) mature, progenitor, precursor and/or dedifferentiated chondrogenic populations for use in cellular therapy strategies.

According to a particular embodiment of the invention, the regulatory cell population and the chondrogenic population for use in the combined population of the present invention are obtained from the same biopsy sample. According to a first embodiment, one fraction of the freshly isolated cells obtained from this biopsy sample is frozen, either directly or within 24 hours of the first expansion, while a second fraction is expanded to obtain a population of expanded chondrocytes (mature chondrocyte population). Alternatively, the regulatory cell population and the chondrocyte population both originate from the same first expansion of a sample of freshly isolated cartilage cells, whereby the regulatory cells correspond to the cell population obtained from the non-adherent cell fraction during the first expansion of the cartilage cells and the mature chondrocyte population corresponds to one or more of the chondrocyte passages of the adherent cell population in the first expansion of those same cartilage cells. Alternatively however, it is envisaged that regulatory populations and chondrocyte or chondrogenic populations are combined originating from different cartilage biopsies or from a cartilage sample which has been divided in a first and a second fraction, whereby a regulatory cell population is optimally isolated from the first fraction and a chondrocyte population is optimally isolated from the second fraction.

According to the present invention, the non-adherent cell population as present within the freshly isolated cell population or obtained during the first period of expansion of a freshly isolated cartilage cell population has regulatory properties which are of particular interest for the generation of cartilage both *in vitro* and *in vivo.* Thus, further aspects of the present invention relate to the use of the identified regulatory cell population in various aspects of cartilage generation and cartilage repair.

The different uses of the regulatory cell population of the present invention are based on its ability to modulate, when mixed with a second cell population, the chondrocyte phenotypic stability of that second cell population. Chondrocyte phenotypic stability of a cell population can be assayed by implanting the population into a nude mouse model as described in WO0124833. Typically, a population of about 5x10⁶ chondrogenic cells of an early passage is capable of producing an implant of mature cartilage within 2-3 weeks. As demonstrated in WO0124833, molecular markers can be used to predict the outcome of such a transplantation, and thus these molecular markers are indicative of the chondrocyte phenotypic stability of a cell population. Typically, the presence of one or more positive markers (such as BMP-2 and/or FGFR-3) and the absence of one or more negative markers (such as ALK-1) can be used to identify a chondrocyte phenotypically stable population.

According to one aspect, the present invention provides methods for maintaining, improving and/or restoring the chondrocyte phenotypic stability of a chondrogenic population. In the methods of the present invention, the regulatory cell population is combined or mixed with a population of chondrogenic cells. The population of chondrogenic cells envisaged in the context of the present invention can be one or a mixture of populations selected from: freshly isolated cartilage cells, expanded passaged chondrocytes, mesenchymal stem cells (MSC), or precursors committed to the osteochondral lineage. In the methods of the present invention, the cells of the regulatory cell population are combined with a chondrogenic cell population whereby the relative amount of the regulatory cells is generally within the range of 1 to 75 %, more particularly within the range of 10 to 50% of the total amount of cells in the mixture. According to the present invention, the regulatory cell population can be combined with a chondrogenic population either in a mixture (cell suspensions are gently mixed to ensure complete mixing of the two cell populations) or in form of so-called chondrospheres, which represent clusters of cartilage-forming cells.

Thus, the present invention provides methods for improving, restoring and maintaining the chondrocyte phenotypic stability of freshly isolated cartilage cells, expanded passaged chondrocytes, mesenchymal stem cells, or precursors committed to the osteochondral lineage. More particularly, the present invention provides methods for improving, restoring and maintaining the chondrocyte phenotypic stability of cultivated chondrocytes, whereby the cultivation can comprise 6 to 10 subsequent passages or more.

The methods of the present invention thus combine, optionally within a composition, a population of regulatory cells (either within a freshly isolated cell population or as an enriched population) with a population of chondrogenic cells. Typically, when the resulting combined cell population is to be used for transplantation, both cell populations will be autologous. Alternatively, the use of e.g. heterologous chondrocytes or heterologous MSC combined with an autologous or even allogeneic cell population of regulatory cells is also envisaged.

A particular aspect of the present invention relates to a method for obtaining, from a cartilage biopsy, a population of cells with improved chondrocyte phenotypic stability. The improvement envisaged by the methods of the present invention can be in different ways but results in an efficient *in vitro* and *in vivo* production of cartilage and/or reconstitution of cartilage defects. It has been established that contacting a population of chondrocytes obtained from a standard monolayer culture with the regulatory cell population of the present invention results in a significant improvement of the chondrocyte phenotypic stability of the resulting cell population, which is more than an additive effect of both cell populations. This positive effect on chondrocyte phenotypic stability has been observed not only for chondrocyte populations of first or second passages, but also for cells which have undergone a high number of passages, which are known to show signs of dedifferentiation. Thus, according to this aspect of the invention methods are provided for obtaining an improved chondrogenic population, such as, for instance for ACT. These methods are based on the combination of two cell populations, the first population being a population of mature chondrocytes, obtained by standard cultivation methods from a cartilage biopsy and the second population being a population of regulatory cells, which can be obtained as described above. Both populations can be independently processed and combined at any stage. The regulatory effect of the regulatory cell population on the chondrocyte population makes it possible to significantly propagate the mature chondrocyte population without affecting the chondrocyte phenotypic stability of the final cellular therapeutic.

Typically, in order to ensure the quality of every 'batch' of chondrocytes that is generated by combining the cell populations according to the present invention, it is subjected to a quality control. This can be a direct assessment of their chondrocyte phenotypic stability either *in vivo* (e.g. nude mouse assay) or *in vitro* or can be based on a validated marker profile (i.e. identification of markers demonstrated to be associated with chondrocyte phenotypic stability, (see WO0124833)). According to the present invention, the combination of the regulatory cell population with a chondrogenic population, such as a classical mature chondrocyte population (even those that have been extensively passaged), will result in an increased chondrocyte phenotypic stability of the resulting combined population, thereby minimizing drop-out rates based on quality assessment. Thus, according to this aspect of the invention, a method is provided for obtaining an improved product for ACT, which not only provides a higher number (for example >10 million cells for P4 chondrocytes) of chondrogenic cells for implantation but also ensures chondrocyte phenotypic stability of the cell population to be used for transplantation.

A further particular embodiment of the present invention relates to the use of the regulatory cell population (either as present in a freshly isolated cartilage population or as an enriched regulatory cell population) to restore the chondrocyte phenotypic stability of a cell population which has a lower or reduced chondrocyte phenotypic stability. While primary cells derived directly from explant tissue (e.g. cartilage biopsy) remain capable of producing and secreting extracellular components characteristic of natural cartilage, specifically type II collagen and sulphated proteoglycans, it is well known that during *in vitro* expansion as monolayers, mature chondrocytes dedifferentiate and lose their ability to form hyaline cartilage *in vivo*. The regulatory cell population of the present invention is capable of improving or restoring the chondrocyte phenotypic stability of cells which have lost their stable phenotype further to extensive passaging. Additionally or alternatively the regulatory cell population of the invention is capable of improving or restoring the chondrocyte phenotypic stability of cartilage cells which, as a result of disease (e.g. osteoarthritic cartilage) or other conditions, do not have or have reduced chondrocyte phenotypic stability. Upon combination of such a cell population with a regulatory cell population according to the present invention, chondrocyte phenotypic stability of the resulting population is restored. This is of interest, e.g. for transplantation purposes, as it places less restrictions on the tissues that can be used as a source of cells for autologous repair strategies such as ACT, or in allogeneic repair strategies.

Yet a further particular embodiment of the present invention relates to the use of the regulatory cell population to direct precursor cells of chondrocytes towards cartilage-forming chondrocytes. More particularly, in the context of the present invention, precursor cells such as mesenchymal stem cells (MSCs) or chondrocyte precursor cells, such as the CDMP-1 positive precursors described in WO0125402 are envisaged. Indeed, it is demonstrated herein that in combination with the regulatory cell population of the present invention, stem cells, more particularly MSCs or precursor cells of the osteochondral lineage are directed towards a cartilage-producing phenotype. Mesenchymal stem cells (MSCs) are capable of differentiating into different mesenchymal lineages, including adipose and connective tissue, bone, and cartilage. MSCs have been identified in human postnatal bone marrow (BM), in peripheral blood, periosteum, muscle, adipose tissue, and connective tissue of human adults. A number of fetal tissues have also been demonstrated to comprise MSCs, such as human first-trimester fetal BM (bone marrow), liver, and blood and second-trimester BM, liver, lung, spleen, pancreas, and kidney. For clinical use, human adult BM is the most common source of MSCs to date. The ability of the regulatory cell population of the present invention to ensure the differentiation of the multipotent MSCs into the chondrocyte cell lineage, and more particularly to induce a cartilage-like expression pattern allows the use of MSCs for an efficient production and/or regeneration of cartilage. Moreover, mesenchymal stem cells have the important advantage that they are a self-renewing population which modulates the immune system and reduces the degree of allogeneic rejection in humans. Thus, they have important potential in the field of regenerative medicine, as they make it possible to use HLA-mismatched (allogeneic) cells to repair or replace damaged tissue. The present invention thus provides methods for preparing a cell population, which comprises combining the regulatory cell population of the present invention with autologous or allogeneic MSCs.

It will be understood that the above-described methods for improving, inducing or restoring chondrocyte phenotypic stability of a chondrocyte cell population or a population precursor cells of chondrocytes can be achieved in different ways. In a particular embodiment the cell population of regulatory cells of the invention is contacted with the other cell population prior to administration to the patient. In one embodiment, the cell populations are contacted within 1-30 minutes before administration, but contacting times of up to 1hr or more are also envisaged. Optionally, the cell populations are contacted as a cell suspension. Alternatively, they are provided together on a matrix for implantation.

A further aspect of the present invention relates to the *in vitro* production of cartilage, using the regulatory cells of the present invention, optionally in combination with one or more populations of chondrogenic cells.

Indeed, in the context of cartilage repair it may be of interest to form cartilage material *ex vivo*, which can be implanted subsequently into the cartilage defect. Such synthetic cartilage material has the advantage that its production can be monitored, through morphological, biochemical, and molecular characterisation, prior to implantation. The regulatory cell population of the present invention can be used either alone, or in combination with a chondrogenic cell population (selected from freshly isolated cartilage cells, expanded and optionally passaged chondrocytes, MSCs) for the expansion of chondrogenic cells in either an anchorage-dependent or an anchorage-independent culture system. In the latter, cells of the regulatory cell population optionally in combination with e.g. chondrocytes can be cultured as colonies within an agarose gel. Alternatively, in another anchorage-independent method, the regulatory cell population optionally in combination with e.g. chondrocytes can be cultured as colonies in suspension culture. In the anchorage-dependent method, the regulatory cell population of the present invention is combined e.g. with a population of chondrogenic cells isolated from primary tissue and grown as monolayer in a two- or three-dimensional matrix to produce cartilage-forming nodules.

In a further aspect, the present invention provides a population of regulatory cells for use as a medicament, as well as methods of treatment comprising the administration of the regulatory cell population to a patient in need thereof. More particularly, the population of regulatory cells is envisaged to be of therapeutic value for the treatment of osteochondral defects, whereby the regulatory cells are administered directly to the defect where cartilage production is needed. Typically the regulatory cells of the present invention will be administered to a zone comprising chondrocytes, most typically chondrocytes which as a result of disease or mechanical defect are no longer capable of producing sufficient amounts of stable hyaline cartilage. According to this aspect of the invention, improvement or restoration of the chondrocyte phenotypic stability of a cell population by the regulatory population of the invention occurs *in situ*, after local implantation into a chondral defect. Again, the cells of the present invention can be administered as cell suspensions or provided on a solid support, such as a matrix.

According to another aspect, the present invention provides compositions, i.e. mixtures or combinations of one or more populations of regulatory cells (either within a freshly isolated cell population or in an 'enriched form') and one or more populations of chondrogenic cells selected from the group consisting of freshly isolated cartilage cells, passaged mature chondrocytes, mesenchymal stem cells or progenitor cells which are committed to the osteochondral cell lineage. As indicated above, the chondrogenic cells used in combination with the regulatory cells of the present invention can be either characterised by a stable chondrocyte phenotype or an unstable phenotype. These mixtures or combinations can be used as a medicament. The present invention further provides pharmaceutical compositions comprising the mixtures and combination of the present invention.

Accordingly, the invention provides methods for the treatment of a cartilage defect by administering the combinations or mixtures of the present invention and the corresponding use of the combinations or mixtures of the present invention in the manufacture of a medicament for the treatment of cartilage defects.

The methods of treatment involving the administration of the regulatory cells of the present invention either alone or in a combination or mixture with another cell population include, but are not limited to, methods of treatment of articular cartilage and meniscal cartilage of joints such as the knee, other treatments of elastic cartilage, fibrocartilage or articular cartilage at other location in the body (e.g. repair of invertebral disks, treatment of osteoarthritis or rheumatoid arthritis). Typically, when used in the treatment of a cartilage defect, the cell populations or combinations of the present invention are injected under a periosteal flap sutured to cover the cartilage defect. Alternatively, the cell populations or combinations of the present invention are used to impregnate a synthetic carrier matrix, or are seeded on native biopolymers and then implanted into the cartilage defect. A variety of biomaterials have been used to date and include three-dimensional collagen gels (e.g. U.S. Pat. No. 4,846,835), reconstituted fibrin-thrombin gels (e.g. U.S. Pat. Nos. 4,642,120; 5,053,050 and 4,904,259), synthetic polymer matrices containing polyanhydride, polyorthoester, polyglycolic acid and copolymers thereof (U.S. Pat. No. 5,041,138), and hyaluronic acid-based polymers.

In addition to the one or more cell populations, the pharmaceutical composition described in the context of the present invention usually includes at least a pharmaceutically acceptable carrier, well known to those skilled in the art and for instance selected from proteins such as collagen or gelatine, carbohydrates such as starch, polysaccharides, sugars (dextrose, glucose and sucrose), cellulose derivatives like sodium or calcium carboxymethylcellulose, hydroxypropyl cellulose or hydroxypropylmethyl cellulose, pregelatinized starches, pectin agar, carrageenan, clays, hydrophilic gums (acacia gum, guar gum, arabic gum and xanthan gum), alginic acid, alginates, hyaluronic acid, collagen, polyglycolic and polylactic acid, dextran, pectins, synthetic polymers such as water-soluble acrylic polymer or polyvinylpyrrolidone, proteoglycans, calcium phosphate and the like.

Additionally or alternatively, the pharmaceutical compositions comprising the regulatory cell populations or combined populations of the present invention further comprise factors such as chemotactic factors or differentiating factors etc.

### BRIEF DESCRIPTION OF THE FIGURES:

The following Examples, not intended to limit the invention to specific embodiments described, may be understood in conjunction with the accompanying Figures,incorporated herein by reference, in which:

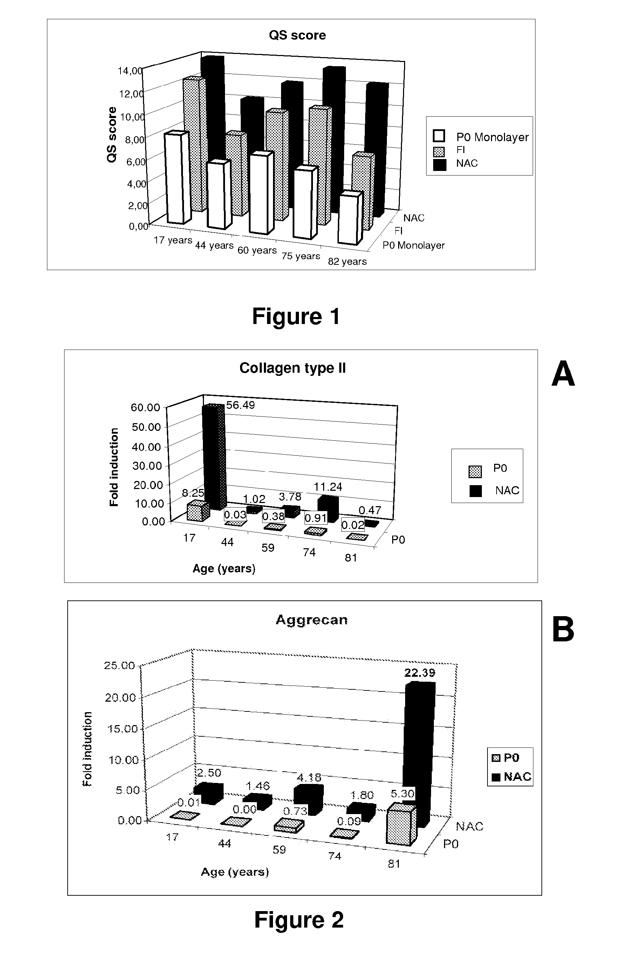

**Figure 1** shows the marker profile (QS score) of freshly isolated cartilage cells (FI), expanded chondrocytes in monolayer (P0) and the regulatory cell population of the present invention (NAC) of biopsies of different individuals. QS scores are defined by a quantitative assessment of expression levels of distinct genes by RT-PCR. High scores correlate with increased chondrocyte phenotypic stability.

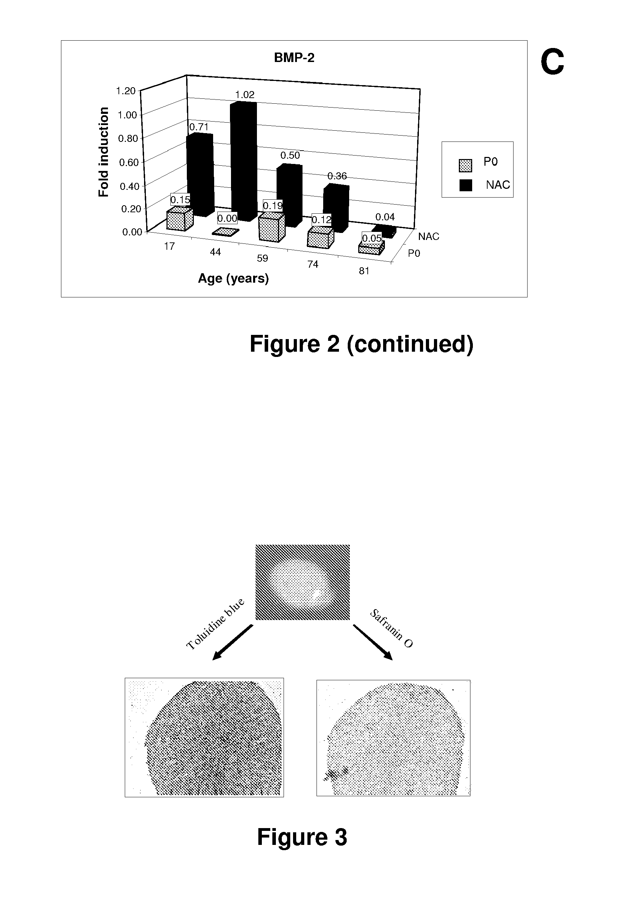

**Figure 2** shows the change in expression of some markers in expanded chondrocytes in monolayer (P0) and in the non-adherent regulatory cell population of the present invention (NAC) of biopsies of different individuals compared to freshly isolated cartilage cells. The markers depicted are Collagen type II (panel A), Aggrecan (panel B) and BMP-2 (panel C).
**Figure 3** shows *in vitro* cartilage formation by the regulatory cell population.

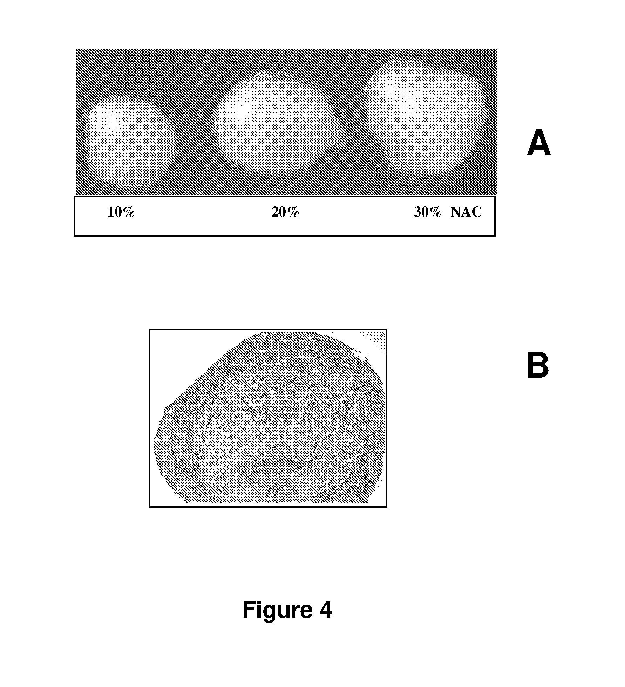

**Figure 4** shows *in vitro* cartilage formation of mixed pellet cultures of chondrocytes admixed with different amounts of the regulatory cell population of the present invention (panel A). Panel A shows pellet formation of a dedifferentiated chondrocyte sample comprising 10, 20 or 30% of the (enriched) non-adherent regulatory cell population (NAC), demonstrating a significant increase in size and mechanical stability with increasing amount of regulatory cells of the present invention. Panel B demonstrates efficient extracellular matrix formation of a TGFβ-stimulated cell pellet consisting of P4 chondrocytes admixed with 50% of regulatory chondrocytes of the present invention as documented by positive safranin O staining.

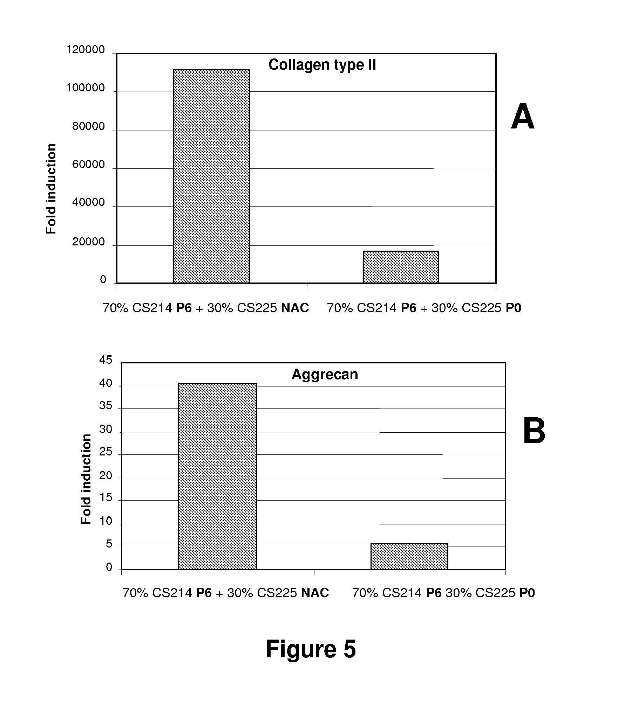

**Figure 5** shows the expression profile for Collagen type II (panel A) and Aggrecan (panel B) of TGFβ-stimulated pellet cultures of P6 chondrocytes admixed with 30% of the regulatory cell population (NAC)(left) and of P6 chondrocytes admixed with 30% of P0 chondrocytes (right) compared to unstimulated pellet cultures of the same composition.

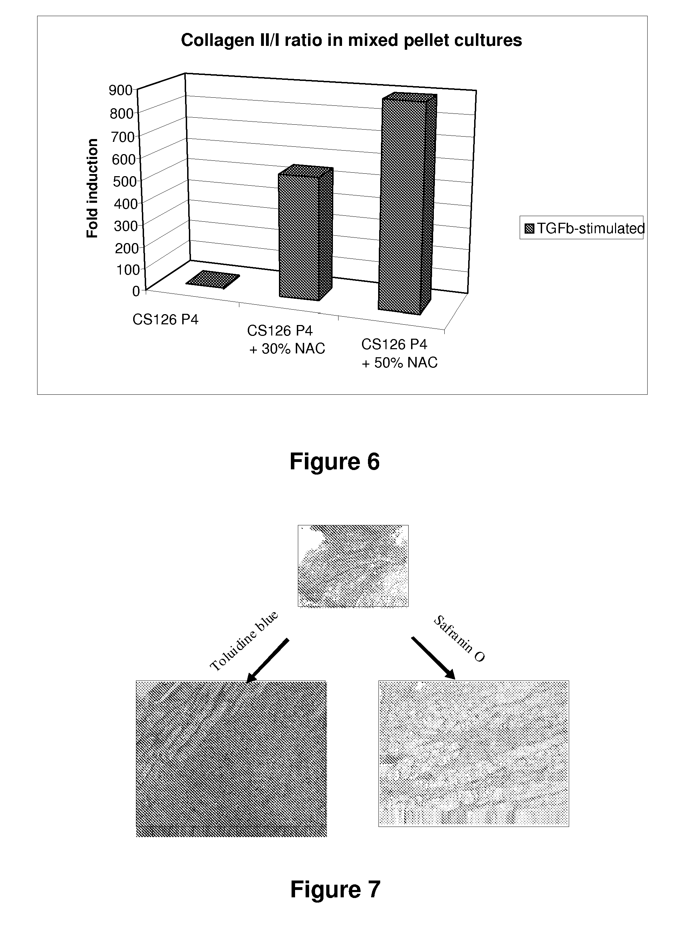

**Figure 6** shows the Collagen type II/I ratio of TGFβ-stimulated pellet cultures of P4 chondrocytes (left), P4 chondrocytes with 30% (middle) or 50% of a regulatory cell population (NAC)(right).
**Figure 7** shows ectopic cartilage formation of dedifferentiated P6 chondrocytes of a 60 year old donor in admixture with 10% of the regulatory cell population.

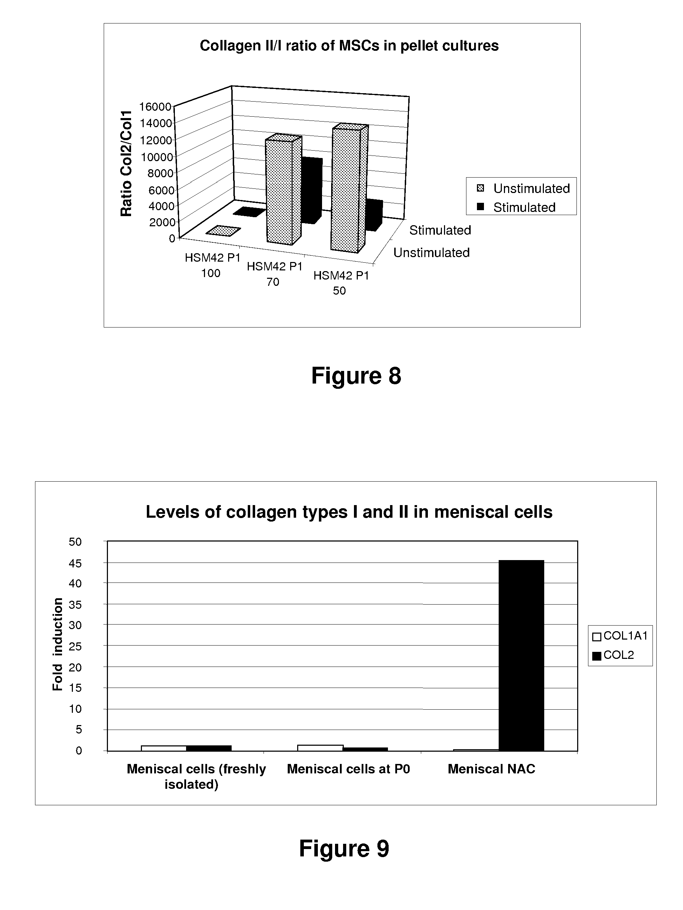

**Figure 8** shows the collagen II/I ratio of pellet cultures consisting of human synovial membrane (HSM)-derived mesenchymal stem cells (MSC) and mesenchymal stem cells admixed with different concentrations of regulatory chondrocytes under TGFβ-stimulated and non-stimulated conditions (the number at each sample indicates the percentage of MSC in a mixture of MSC and a population with regulatory cells).
**Figure 9** shows a comparison of the expression profile for collagen type I and type II of a freshly isolated cell population, a population of adherent mature chondrocytes of P0 and a regulatory cell population (NAC), as obtained from meniscal cartilage, according to one embodiment of the invention.

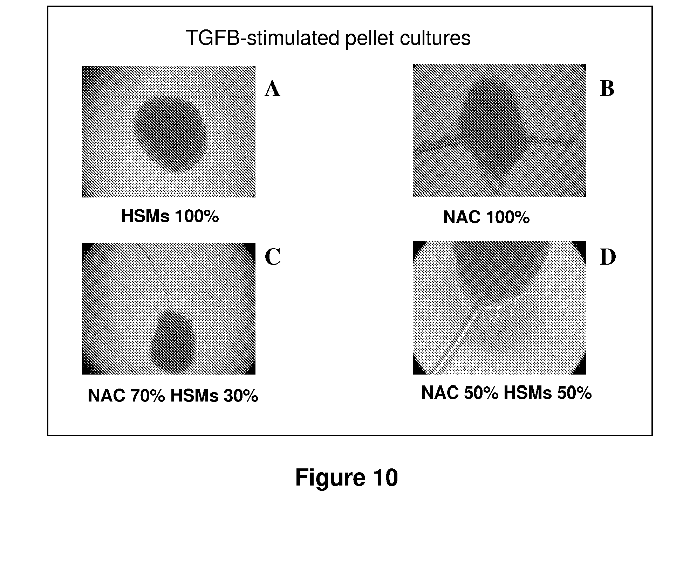

**Figure 10** shows the fiber formation within TGFβ-treated pellet cultures of regulatory cells (NAC) obtained from meniscal cartilage compared to synovial membrane-derived stem cells (HSMs) according to an embodiment of the invention. A: pellet comprising 100% synovial membrane-derived stem cells; B: pellet comprising 100% meniscal regulatory cell population (NAC); C: pellet comprising 70% meniscal regulatory cells (NAC) and 30% synovial membrane-derived stem cells; D: pellet comprising 50% meniscal regulatory cells (NAC) and 50% mesenchymal stem cells (HSMs).

### EXAMPLES

### Example 1: Isolation and characterisation of regulating cells.

Cartilage/meniscal biopsies were minced in serum-free DMEM medium and antibiotics. Subsequently a solution of 0.1% Collagenase A in serum-free DMEM medium and antibiotics was added to the tissue. The enzymatic reaction was performed in a tube in the rotor of an incubator at 37 °C for a period of approximately 12 hours to allow the release of single cells or cell clusters from the biopsy. Cells were collected by centrifugation and subsequently transferred into DMEM medium with serum and antibiotics. The cell culture was performed at 37°C with 5% CO₂.

After 4 days of starting the cell culture, the medium was exchanged as follows: the culture supernatant was harvested and fresh medium was added to the adherent chondrocyte fraction. The loosely attached and non-adherent cells were collected from the cell supernatant by centrifugation at 1500rpm for 10 minutes. These phenotypically stable cells were then either frozen or kept in culture flasks or specifically treated plates or used directly in various experimental procedures/clinical applications (as described below).

In humans this regulatory cell population with round morphology was found to be present in digested cartilage biopsies of different tissue sources including cartilage and meniscus, independent of age or disease. In articular cartilage digests the chondrocyte subpopulation with regulating cells was found to be present at concentrations between 8 and 20% of the amount of cells seeded. (Table 1). In meniscal biopsies the non-adherent cell population was found to be present as about 5-20% of the total cell number.

**Table 1: Percentage of non-adherent cells in total cell population obtained from cartilage digests.**

| Articular chondrocyte biopsy | age | gender | Cell viability | % non-adhering |
|---|---|---|---|---|
| CS228 | 81 | Male | 85% | 14% |
| CC0039 | 25 | n.d | 91% | 20% |
| CC0009 | 23 | Male | 86% | 8.5% |
| CC0044 | | | 89% | 14% |

Upon immunohistological analysis of the non-adhering cell population it was established that at least a fraction of the non-adhering cells have a pericellular matrix consisting of Collagen VI. A flow-cytometric analysis revealed the expression of CD29, CD49e, CD51, CD54 and CD56.

The population of regulatory cells demonstrated an exceptional expression profile of markers which are correlated with cartilage phenotypic stability.

Generally, when articular chondrocytes are cultured in monolayer they lose their characteristic phenotype. The change towards a polygonal or even fibroblastic morphology is accompanied by a dramatic reduction in the expression of cartilage-specific extracellular matrix genes. The non-adhering regulatory cell population not only keeps its round morphology during the first expansion period, but these cells also have an improved expression profile of markers of phenotypically stable cartilage, compared to adhering P0 mature chondrocytes (analysed at the end of the first expansion)(Figure 1). While the presence of regulatory cells in the freshly isolated cartilage cells (FI) ensures an improved expression profile of markers of phenotypically stable cartilage over the adhering P0 mature chondrocytes, the expression of these markers is lower than that of the enriched regulatory cell population (Figure 1).

All analyses demonstrated that, independent of the age of the patient from which the cartilage is retrieved, the regulatory cell population described in the present invention presents higher QC scores compared to chondrocytes from P0 or even to freshly isolated (FI) chondrocytes.

In all but one sample (donor aged 81 with severe OA), the collagen type II expression rates were higher than that of freshly isolated cells (data not shown) and clearly above those of P0 adhering mature chondrocytes (Figure 2A). Expression rates for aggrecan were significantly above those for P0 adhering mature chondrocytes (Figure 2B) and freshly isolated cartilage cells (data not shown). BMP-2 expression rates were found to be higher compared to P0 adhering mature chondrocytes (Figure 2C) but lower than those of freshly isolated cells (data not shown).

### Example 2: Cartilage-forming capacity of the regulating cells of the present invention.

About 200.000 cells of a non-adherent regulatory cell population (biopsy CS225) were centrifuged at 1500 rpm in conical 15 ml tubes and cultured in a pellet under chondrogenesis-inducing conditions. The composition of this medium is as follows: DMEM containing 1:100 Antibiotics, 1:100 Sodium Pyruvate, 1:100 ITS+ [BD Biosciences], 10⁻⁷ M Dexamethasone and 1:1000 37,5 mg/ml Ascorbic Acid. The tube lids are then slightly loosened and cell culture is performed at 37°C with 5% CO2. The cell culture period is 2 weeks, with media changed every 2-3 days. TGF-beta was also added to some pellets. After 2 weeks, the pellets were either used for histology or RNA was extracted for real-time quantitative PCR.

TGFβ stimulation resulted in mechanically stable cartilaginous tissue of exceptional quality as documented by metachromatic stainings of the extracellular matrix of sections of paraffin embedded cartilage with toluidine blue and safranin O (Figure 3).

These data demonstrate that the regulatory cell population of the present invention is in itself a phenotypically stable chondrocyte population, capable of producing high quality cartilage.

### Example 3: Ability of the regulatory cell population to improve chondrocyte phenotypic stability of mature chondrocytes

Mixed pellet culture was performed similar to the procedure described above in Example 2, but using a combination of regulatory cells and mature chondrocytes.

TGFβ-stimulated mixed pellet cultures of repeatedly passaged chondrocytes and differing amounts (10%, 20%, 30%) of the regulatory cell population of the present invention (NAC) observed after two weeks, displayed a stronger cartilage formation capacity compared to dedifferentiated chondrocytes alone, as can be seen by an increase in pellet size (Figure 4A) and a metachromatic staining for safranin O (Figure 4B).

The combination of the regulatory cell population of the present invention and 70% P6 chondrocytes induced a high quality extracellular matrix. A significant increase in the expression of collagen type II and aggrecan was observed in mixed pellet cultures containing regulatory chondrocytes compared to mixed pellet cultures using a combination of P0 chondrocytes and P6 chondrocytes (Figure 5).

The improved cartilage-forming capacity was also reflected by a significant increase in the collagen type II: type I ratio. (Figure 6). Next to an increased expression rate for collagen type II a decrease in the expression of collagen type I was observed (Figure 6, middle and right bar). In contrast, pellet cultures of solely dedifferentiated P4 chondrocytes did not form mechanically stable cartilage and demonstrated only a limited capacity to regain expression rates for collagen type II in 3D culture (Figure 6, left).

### Example 4: Generation of cartilage with phenotypic stability in muscular tissue of nude mice.

The redifferentiation capacity of the regulatory cell population of the present invention was demonstrated by ectopic cartilage formation after intramuscular injection in nude mice. In this method about 3 to 4 million cells are mixed with HBSS medium and injected in a volume of 50 microliter in the thigh of a nude mouse. Animals were sacrificed after 2-3 weeks and the thigh muscle was formalin-fixed, embedded in wax and cut in section for histology.

When injecting 4 million cells consisting of a combination of 90% P6 chondrocytes and 10% of P0 mature adhering chondrocytes, no cartilage implant was detectable. However, when injecting the same number of P6 chondrocytes in combination with 10% regulating non-adherent cells (CS225-regulating cells/CS214P6) a huge cartilage implant was formed.

Metachromatic stainings with Toluidine blue as well as safranin O demonstrated the excellent quality of this hyaline cartilage-like implant (Figure 7).

### Example 5: Differentiation of Mesenchymal Stem Cells into the chondrogenic lineage.

Mesenchymal stem cells were combined with different amounts of regulating cells and kept in TGFβ-stimulated mixed pellet cultures as described above or used *in vivo* in the nude mouse assay as described above. Differentiation into the chondrogenic lineage was dramatically improved by culturing a mixture of 70% mesenchymal stem cells and 30% of regulatory chondrocytes. The quality of cartilage pellets was evaluated by measuring the Collagen II versus Collagen I expression (Figure 8B). The results show that regulating cells are superior in promoting differentiation into cartilage by boosting the collagen type II expression to a significantly higher degree than the most commonly used differentiation agent TGFβ alone.

### Example 6: Treatment of OA joints by a combined cellular product consisting of autologous or allogeneic non-hematopoietic stem cells and autologous or allogeneic phenotypically stable chondrocytes.

Autologous or allogeneic non-adherent cells harvested according to Example 1 are combined with expanded mature chondrocytes, human synovial membrane-derived stem cells, or another source of autologous non-hematopoietic stem cells. The combination of the non-adherent regulatory cells with the chondrocyte progenitor cells results in a cellular product with combined therapeutic activity, whereby the regulatory cells direct the stem cells towards chondrogenic lineage, while the stem cells can modulate an inflammatory reaction. This combined cellular product, capable of regenerating cartilage and modulating inflammation is of particular interest e.g. in the treatment of cartilage defects in rheumatoid arthritis.

### Example 7: Isolation and characterisation of a regulatory cell population from meniscal cartilage.

A regulatory cell population was enriched from a sample of meniscal cartilage by collecting the non-adhering fraction as described in Example 1. The expression profile of the regulatory cell population was determined. The regulatory cell population obtained from meniscal cartilage is characterized by a significantly higher expression rate for type II collagen, compared to the corresponding population of adhering P0 chondrocytes obtained from the meniscal cartilage sample (Figure 9). The expression of type II collagen by freshly isolated meniscal cartilage cells was found to be low. In contrast the expression rate for type I collagen is reduced in the enriched regulatory cell population obtained from meniscal cartilage.

### Example 8: Fiber forming capacity of a regulatory cell population obtained from meniscal cartilage.

A pellet culture was obtained as described in Example 2, using an enriched regulatory cell population obtained from meniscal cartilage. TGFβ-stimulated pellet cultures of the meniscal regulatory population were found to form collagen fibers, indicating that this cell population is capable of integrating into surrounding tissue (Figure 10, upper right panel). Mixed pellet cultures of the meniscal-derived regulatory cell population and synovial membrane-derived stem cells were prepared and stimulated with TGFβ. The presence of collagen fibers was observed with different relative amounts of meniscal regulatory cells (Figure 10, lower panels), while a pellet culture of synovial membrane-derived stem cells alone did not form collagen fibers (Figure 10, upper left panel). This property of meniscal regulatory cells is especially of interest in the repair of inner meniscal tears, for which at current state no treatment option is available (see below).

### Example 9: Treatment of meniscal defects using a regulatory cell population derived from meniscal cartilage

A population of regulatory cells is enriched from a sample of meniscal cartilage using the procedure as described in example 1, and the number of cells is counted. The meniscal-derived regulatory cell population is then mixed with expanded human synovial membrane-derived stem cells, optionally provided in a scaffold, and used in the treatment of meniscal defects, even within the avascular region of the menisci.

## Claims

1. A composition comprising a combination of two or more different cell populations comprising:
a) a cell population comprising regulatory cells which is either (i) a population of freshly isolated cartilage cells obtainable from a cartilage biopsy or (ii) a population of enriched regulatory cells obtainable by the collection of non-adherent cells from the supernatant of a P0 culture of freshly isolated cartilage cells obtained from a cartilage biopsy, and
b) one or more cell populations selected from the group consisting of a passaged chondrocyte cell population, a population of mesenchymal stem cells, a population of chondrocyte precursor cells.

2. The composition of claim 1, comprising a population of enriched regulatory cells, wherein the relative amount of said enriched regulatory cells is within the range of 1 to 75 % of the total number of cells in the combination.

3. The composition of claim 1 or 2, wherein said population comprising regulatory cells is obtained from articular cartilage.

4. The composition of claim 1 or 2, wherein said population comprising regulatory cells is obtained from meniscal cartilage.

5. A combination of two or more different cell populations according to any one of claims 1 to 4 for use as a medicament.

6. A combination of two or more different cell populations according to any one of claims 1 to 4 for the treatment of cartilage defects.

7. A scaffold comprising the combination of two or more different cell populations according to any one of claims 1 to 4.

8. Use of a population of non-adhering, non-passaged regulatory cells, for improving, maintaining or restoring the chondrocyte phenotypic stability of an expanded or passaged chondrocyte population *in vitro.*

9. Use of a population of non-adhering regulatory cells, for differentiating mesenchymal stem cells into the chondrogenic lineage *in vitro.*

10. A population of non-adhering, non-passaged regulatory cells, for use as a medicament,
wherein said population is obtainable by a method comprising the steps of:
a) mechanically and/or enzymatically treating an isolated cartilage sample to obtain individual cells,
b) transferring said individual cells obtained under (a) to a cell cultivation recipient to allow the expansion of chondrocytes in a monolayer,
c) maintaining said recipient in adequate cell cultivation conditions so as to obtain a population of adherent cells and a supernatant comprising a population of non-adherent cells,
d) collecting from said recipient, after at least 2 days and/or upon at least about 30% confluency, said supernatant comprising said population of non-adherent cells, and
e) collecting from said supernatant said population of non-adherent cells, corresponding to said regulatory cell population.

11. A population of non-adhering, non-passaged regulatory for treating a cartilage defect,
wherein said population is obtainable by a method comprising the steps of:
a) mechanically and/or enzymatically treating an isolated cartilage sample to obtain individual cells,
b) transferring said individual cells obtained under (a) to a cell cultivation recipient to allow the expansion of chondrocytes in a monolayer,
c) maintaining said recipient in adequate cell cultivation conditions so as to obtain a population of adherent cells and a supernatant comprising a population of non-adherent cells,
d) collecting from said recipient, after at least 2 days and/or upon at least about 30% confluency, said supernatant comprising said population of non-adherent cells, and
e) collecting from said supernatant said population of non-adherent cells, corresponding to said regulatory cell population.
